# EUROPEAN PATENT APPLICATION

(11) **EP 3 693 364 A1**
(43) Date of publication of application: **12.08.2020**
(21) Application number: 19156491.3
(22) Date of filing: 11.02.2019
(51) Int. Cl.: C07D 401/04, A61P 7/06, A61K 31/4439

(54) **CRYSTALLINE SALTS OF A HEMOGLOBIN S ALLOSTERIC MODULATOR**

(71) Applicant: SANDOZ AG, 4056 Basel (CH)
(72) Inventor: The designation of the inventor has not yet been filed
(74) Representative: Prüfer & Partner mbB Patentanwälte · Rechtsanwälte

(57) **Abstract**

The present invention relates to new crystalline forms of 2-hydroxy-6-((2-(1-isopropyl-1H-pyrazol-5-yl)pyridin-3-yl)methoxy)benzaldehyde (also referred to as "voxelotor"), in particular to crystalline salts of voxelotor with 1,5-naphthalenedisulfonic acid (also referred to as "voxelotor napadisylate" salts) and methods for preparing same. The invention also refers to a pharmaceutical composition comprising the said voxelotor napadisylate salts and one or more pharmaceutically acceptable excipients. The pharmaceutical composition of the present invention can be used as a medicament for increasing oxygen affinity of hemoglobin S, in particular for the treatment of oxygen deficiency associated with stickle cell anemia.

## Description

### Field of the invention

The present invention relates to new crystalline forms of 2-hydroxy-6-((2-(1-isopropyl-1H-pyrazol-5-yl)pyridin-3-yl)methoxy)benzaldehyde (also referred to as "voxelotor"), in particular to crystalline salts of voxelotor with 1,5-naphthalenedisulfonic acid (also referred to as "voxelotor napadisylate" salts) and methods for preparing same. The invention also refers to a pharmaceutical composition comprising the said voxelotor napadisylate salts and one or more pharmaceutically acceptable excipients. The pharmaceutical composition of the present invention can be used as a medicament for increasing oxygen affinity of hemoglobin S, in particular for the treatment of oxygen deficiency associated with stickle cell anemia.

### Background of the invention

Voxelotor, also known under the chemical name 2-hydroxy-6-((2-(1-isopropyl-1H-pyrazol-5-yl)pyridin-3-yl)methoxy)benzaldehyde, can be represented by the following chemical structure according to Formula (A):

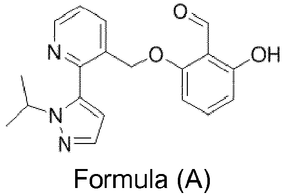

Voxelotor is an orally bioavailable modulator and stabilizer of sickle cell hemoglobin (HbS), with potential use in the treatment of sickle cell disease (SCD). Upon administration, voxelotor targets and covalently binds to the N-terminal valine of the alpha chain of HbS. This stabilizes HbS, thereby improving oxygen binding affinity. The binding of voxelotor to HbS prevents HbS polymerization, reduces sickling, decreases red blood cell (RBC) damage and increases the half-life of RBCs. This improves blood flow and decreases hemolytic anemia.

WO2013/102142 A1 discloses substituted benzaldehyde compounds used for increasing tissues oxygenation, including voxelotor (compound 43). Examples 17 and 18 describe the synthesis of voxelotor, which is isolated after chromatographic purification as a pale yellow oil or a pale yellow solid, respectively, and is characterized by NMR spectroscopy and mass spectrometry. No further data regarding solid state are given.

WO2015/031284 A1 provides pharmaceutical formulations for the allosteric modulation of hemoglobin (S) and methods for their use in treating disorders mediated by hemoglobin (S) and disorders that would benefit from tissue and/or cellular oxygenation. The invention claims that the said pharmaceutical formulations comprise a hemoglobin S allosteric modulator (for example voxelotor) and at least one pharmaceutically acceptable wetting agent. This indicates that the wetting properties of voxelotor is low and that it is an issue for the preparation of pharmaceutical compositions.

WO2015/031285 A1 discloses crystalline salts of voxelotor and in particular crystalline hydrochloride salts designated as Form I, Form II and Form III. Form I is described as an ansolvate salt, whereas Form II and Form III are solvated crystalline hydrochloride salts of voxelotor. Moreover, WO2015031285 A1 reported that the hydrochloride ansolvate Form I has superior stability and other physicochemical properties compared to crystalline hydrochloride Form II and Form III.

According to WO2015/120133 A1, hydrochloride salt of voxelotor is not stable as it readily disproportionates or loses hydrochloric acid, yielding voxelotor free base. For example, partial conversion to the free base was observed within 12 hours at 30°C. Moreover, it is mentioned that sulfate salt of voxelotor also disproportionates from certain solvents when precipitated with water and that voxelotor free base provides therefore a more stable chemical entity compared with the corresponding hydrochloride or sulfate and such other salt. WO2015/120133 A1 also discloses crystalline forms of voxelotor (free base) and more particularly the crystalline free base ansolvate forms designated as Form I, Form II and Material N, as well as the crystalline free base solvates designated as Form E, Form F, Form G, Form H, Form J, Form K, Form L, Form M, Form O and Form P. The ansolvates Form I, Form II and Material N are described as anhydrous forms, characterized by having an endothermic peak between (95±2)°C and (97±2)°C as measured by differential calorimetry. According to WO2015/120133 A1, free base Form I is a metastable phase. It generally nucleates first from a slurry and transforms to free base Form II, a thermodynamically more stable phase relative to Form I, by extending the slurry time. Free base Material N is reported as being stable relative to Form I and Form II at room temperature and is enantiotropically related to Form II with a specific transition temperature estimated to be near 40°C. At operating temperatures above this transition temperature, i.e. above 40°C (e.g., at 50°C) free base Form II appears to be the most stable form, relative to Form I and Material N, and voxelotor exists primarily as Form II, which may contain some residual Material N. Under operating temperatures below this transition temperature, i.e. below 40°C (e.g. at 30°C) voxelotor exists primarily as Material N, which may contain some residual Form II. Furthermore, the crystalline free base solvates (i.e. Form E, Form F, Form G, Form H, Form J, Form K, Form L, Form M, Form O and Form P) are described as possible intermediates fort he preparation of the crystalline free base ansolvate forms of voxelotor, as they desolvate to voxelotor free base, for example by subjecting them to vacuum conditions.

WO2018/071678 A1 discloses high drug load pharmaceutical compositions comprising from about 30% to about 70% by weight of voxelotor, including Form II (free base), and a microcrystalline cellulose as a filler provided that at least one microcrystalline cellulose is a high-compactable microcrystalline cellulose and wherein the percent by weight is relative to the total weight of the tablet. Moreover, the pharmaceutical composition comprises from about 20% to about 40% by weight of the high-compactable microcrystalline cellulose. Figure 1 of WO2018/071678 A1 shows that the tablets of voxelotor Form II containing surfactant (i.e. sodium lauryl sulfate; G2 formulations) have a better dissolution profile than tablets without surfactant (G1 formulation and capsules). These results fit with the information provided in WO2015/031284 A1, in which pharmaceutical formulations comprising a hemoglobin S allosteric modulator, such as voxelotor, and at least one pharmaceutically acceptable wetting agent are reported. This indicates that the wetting properties of voxelotor free base is an issue for the preparation of pharmaceutical compositions.

As reported above, crystalline Form I of voxelotor is a metastable polymorph. Furthermore, it is difficult to prepare Form II or Material N of voxelotor in pure polymorphic form. These two anhydrous forms are enantiotropically related with a specific transition temperature estimated to be near 40°C: voxelotor exists primarily as Form II at operating temperatures above 40°C (e.g. at 50°C) and as Material N below 40°C (e.g. at 30°C). The physical properties of Form II and Material N are therefore not consistent at a range of temperature, which is typical of the regular working conditions encountered during crystalline form manufacturing and finished dosage form preparation. Moreover, crystalline Form II of voxelotor, which is used for the preparation of pharmaceutical composition, as reported in WO2018/071678 A1, has a low wettability that has to be compensated by the use of a surfactant and a high loading of microcrystalline cellulose in the pharmaceutical formulation.

There remains thus a need for further crystalline form of voxelotor suitable for the development of pharmaceutical dosage forms, preferably crystalline form with improved properties, and which can be prepared with a reliable industrial process and retains its physicochemical properties over time and among various manufactured batches. There is a particular need for thermodynamically stable forms of voxelotor with superior wetting properties and improved physicochemical behavior at a range of temperature that is typical of the regular working conditions encountered during crystallization process and finished dosage form preparation.

### Summary of the invention

The present invention relates to crystalline salts of voxelotor with 1,5-naphthalenedisulfonic acid, 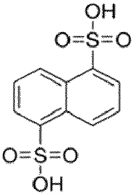 also referred to as "voxelotor napadisylate" salts, and methods for preparing same. More specifically, it relates to crystalline voxelotor heminapadisylate salts, and in particular to crystalline Form A, Form B, Form C, and Form D. The invention also refers to a pharmaceutical composition comprising crystalline salts of voxelotor of the present invention and one or more pharmaceutically acceptable excipients. In addition, the present invention relates to the use of said pharmaceutical composition as a medicament for increasing oxygen affinity of hemoglobin S, in particular for the treatment of oxygen deficiency associated with stickle cell anemia.

The crystalline salts of voxelotor of the present invention have the advantages as demonstrated in the experimental section herein and as further disclosed elsewhere herein.

### Definitions

As used herein the term "room temperature" refers to a temperature in the range from 20 to 30 °C, preferably to a temperature in the range from 22 to 27 °C.
As used herein, the term "measured at a temperature in the range from 20 to 30 °C" refers to a measurement under standard conditions. Typically, standard conditions mean a temperature in the range from 20 to 30 °C, i.e. at room temperature. A preferred temperature for measurements is 22 °C. Typically, standard conditions additionally mean a measurement at 20% to 75% RH (relative humidity), with about 25% to 40% RH being a preferred controlled humidity value for a measurement.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 103 to 1020 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in peak positions and relative intensities of the peaks need to be taken into account. For example, a typical precision/accuracy of the 2-Theta values is in the range of ± 0.2° 2-Theta, preferably in the range of ± 0.1° 2-Theta. Thus, a diffraction peak that usually appears at 15.0° 2-Theta for example can appear between 14.8° and 15.2° 2-Theta, preferably between 14.9° and 15.1° 2-Theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

A salt of voxelotor may be referred to herein as being characterized by graphical data "as shown in" a figure. Such data include, for example, powder X-ray diffractograms. The person skilled in the art understands that factors such as variations in instrument type, response and variations in sample directionality, sample concentration and sample purity may lead to small variations for such data when presented in graphical form, for example variations relating to the exact peak positions and intensities.

The term "stable", as used herein, means that the respective crystalline form is maintained in an amount of at least 90% when stored at 40% RH, preferably 80% RH (open conditions) and at a temperature in the range of from 20 to 45°C for 1 week, 2 weeks, preferably 4 weeks, or even 8 weeks. The amount of crystalline form can be determined by PXRD.

As used herein, the term "wetting agent" means a compound used to lower the surface tension between a liquid and a solid, thereby aiding in attaining intimate contact between solid particles and the liquid. Suitable wetting agents may be one or more of anionic, cationic, or non-ionic surface-active agents or surfactants. In some embodiments, non-ionic surfactants having HLB ("hydrophilic-lipophilic balance") value between 7 and 10 are used as wetting agents. Wetting agents may further include one or more of gum acacia, guar gum, xanthan gum, kaolin, bentonite, hectorite, tragacanth, sodium alginate, pectin, and the like. Suitable anionic surfactants may be one or more of sodium dodecyl sulfate (SDS), sodium lauryl sulfate (SLS), sodium laurate, dialkyl sodium sulfosuccinates, sodium stearate, potassium stearate, sodium oleate, and the like.
Suitable cationic surfactants may be one or more of benzalkonium chloride, bis-2- hydroxyethyl oleyl amine, benzethonium chloride, cetrimide, and the like.
Suitable non-ionic surfactants may be one or more of poloxamers, polyoxyethylene sorbitan fatty acid esters, fatty alcohols such as lauryl, cetyl and stearyl alcohols; glyceryl esters such as the naturally occurring mono-, di-, and tri-glycerides; fatty acid esters of fatty alcohols and other alcohols such as propylene glycol, polyethylene glycol, sorbitan, sucrose, cholesterol; polysorbates (20, 60 or 80); Tyloxapol (a nonionic liquid polymer of the alkyl aryl polyether alcohol type, also known as superinone or triton); and the like.
Other useful wetting agents include, by way of example and without limitation, gelatin, casein, lecithin (phosphatides), stearic acid, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene stearates, phosphates, carboxymethylcellulose calcium, carboxymethylcellulose sodium, methylcellulose, hydroxyethylcellulose, hydroxy propylcellulose, hydroxypropylmethylcellulose phthalate, noncrystalline cellulose, magnesium aluminum silicate, triethanolamine, polyvinyl alcohol, and polyvinylpyrrolidone (PVP).

### Abbreviations

- PXRD: powder X-ray diffractogram
- DSC: differential scanning calorimetry
- TGA: thermogravimetric analysis
- GMSD: gravimetric moisture sorption / desorption
- Δm: mass difference
- RH: relative humidity
- wt.%: weight percent
- TGMS: thermogravimetric mass spectroscopy
- DSA: drop shape analysis
- CA(M): average contact angle value based on two contact angles (left and right side of the drop)

### Brief description of the figures (drawings)

Figure 1: Representative PXRD of the crystalline Form A of voxelotor heminapadisylate prepared according to the procedure described in Example 1-2. The x-axis shows the scattering angle in °2-Theta, the y-axis shows the intensity of the scattered X-ray beam in counts of detected photons.
Figure 2: Representative DSC curve of the crystalline Form A of voxelotor heminapadisylate, prepared according to the procedure described in Example 1-3. The X-axis shows the temperature / °C and the Y-axis shows peak heights / Wg-1. Endothermic events are plotted up.
Figure 3: Representative TGA curve of the crystalline Form A of voxelotor heminapadisylate, prepared according to the procedure described in Example 1-3. The X-axis shows the temperature / °C and the Y-axis shows sample mass / %. Endothermic events are plotted up.
Figure 4: Representative PXRD of the crystalline Form B of voxelotor heminapadisylate prepared according to the procedure described in Example 2-2. The x-axis shows the scattering angle in °2-Theta, the y-axis shows the intensity of the scattered X-ray beam in counts of detected photons.
Figure 5: Representative DSC curve of the crystalline Form B of voxelotor heminapadisylate, prepared according to the procedure described in Example 2-2. The X-axis shows the temperature / °C and the Y-axis shows peak heights / Wg-1. Endothermic events are plotted up.
Figure 6: Representative TGA curve of the crystalline Form B of voxelotor heminapadisylate, prepared according to the procedure described in Example 2-2. The X-axis shows the temperature / °C and the Y-axis shows sample mass / %. Endothermic events are plotted up.
Figure 7: Representative PXRD of the crystalline Form C of voxelotor heminapadisylate prepared according to the procedure described in Example 3. The x-axis shows the scattering angle in °2-Theta, the y-axis shows the intensity of the scattered X-ray beam in counts of detected photons.
Figure 8: Representative DSC curve of the crystalline Form C of voxelotor heminapadisylate, prepared according to the procedure described in Example 3. The X-axis shows the temperature / °C and the Y-axis shows peak heights / Wg-1. Endothermic events are plotted up.
Figure 9: Representative TGA curve of the crystalline Form C of voxelotor heminapadisylate, prepared according to the procedure described in Example 3. The X-axis shows the temperature / °C and the Y-axis shows sample mass / %. Endothermic events are plotted up.
Figure 10: Representative PXRD of the crystalline Form D of voxelotor heminapadisylate prepared according to the procedure described in Example 4. The x-axis shows the scattering angle in °2-Theta, the y-axis shows the intensity of the scattered X-ray beam in counts of detected photons.

### Detailed description

Preparation of pure crystalline Form II of voxelotor is difficult as it is known, e.g., from WO2015/120133 A1 and crystalline Form II has a tendency to convert to Material N below 40°C (e.g., at room temperature). Moreover, crystalline free base voxelotor, such as Form II, is characterized by an endothermic peak between (95±2)°C and (97±2)°C as measured by differential calorimetry, corresponding to melting endotherm. This relatively low melting temperature can lead to chemical stability issues when storing or processing free base voxelotor at high temperature conditions. Furthermore, crystalline voxelotor Form II has low wetting properties, which need to be compensated by the use of surfactants and high loading of microcrystalline cellulose in a pharmaceutical formulation to reach adequate dissolution profile.

The present inventors have surprisingly found that voxelotor salts prepared with 1,5-naphthalenedisulfonic acid have superior physicochemical properties (e.g., superior thermal stability and wettability) than crystalline free base forms of voxelotor, such as Form II. This allows the preparation of stable pharmaceutical compositions comprising no wetting agent (i.e. surfactant) and a lower loading of microcrystalline cellulose. The pharmaceutical compositions of the present invention are advantageous compared to pharmaceutical compositions comprising crystalline Form II of voxelotor in that their higher thermal stability can improve storage stability, for example under ambient or high temperature conditions. One objective of the present invention is thus to provide a pharmaceutical composition comprising an effective amount of crystalline voxelotor which retains its physicochemical stability upon storage, for example throughout its shelf life.

Therefore, the present invention relates to crystalline salts of voxelotor with 1,5-naphthalenedisulfonic acid, also referred to as voxelotor napadisylate salts, which are characterized by having a molar ratio of voxelotor and acid in the range of from 4.0 : 1.0 to 1.0 : 4.0, preferably of from 2.0 : 1:0 to 1.0 : 2.0 and most preferably the molar ratio is 2.0 : 1.0. More specifically, it relates to crystalline voxelotor heminapadisylate salts, which are characterized by having a molar ratio of voxelotor and acid of 2.0 : 1.0.

The present inventors have surprisingly found that crystalline salts of voxelotor prepared with 1,5-naphthalenedisulfonic acid, in particular voxelotor napadisylate salts of the present invention, do not disproportionate to voxelotor free base (e.g. upon storage at 40°C und 75% relative humidity), in contrast to the voxelortor salts, such as hydrochloride, sulfate and such other salts, mentioned in WO2015/120133 A1. Additionally, the present inventors have also surprisingly found that the crystalline salts of voxelotor of the present invention, and particularly Form A and Form D, are stable and exhibit improved physicochemical properties (e.g. higher thermal stability, higher wettability) compared to crystalline free base voxelotor, such as crystalline Form II used in for the preparation of pharmaceutical formulations as reported in WO2018/071678 A1. Higher thermal stability can improve storage stability, for example under high temperature conditions or for a longer storage period.

### Voxelotor heminapadisylate Form A

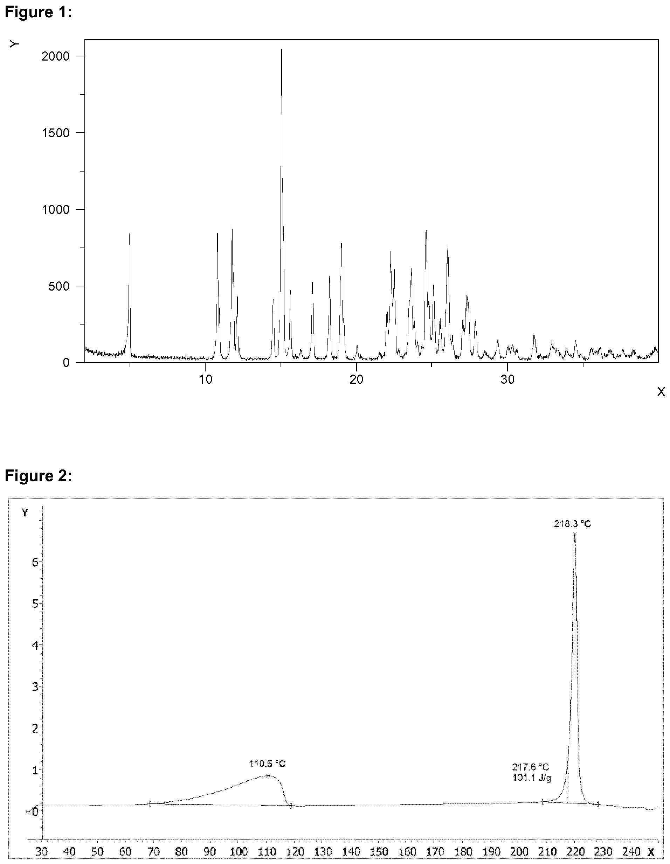

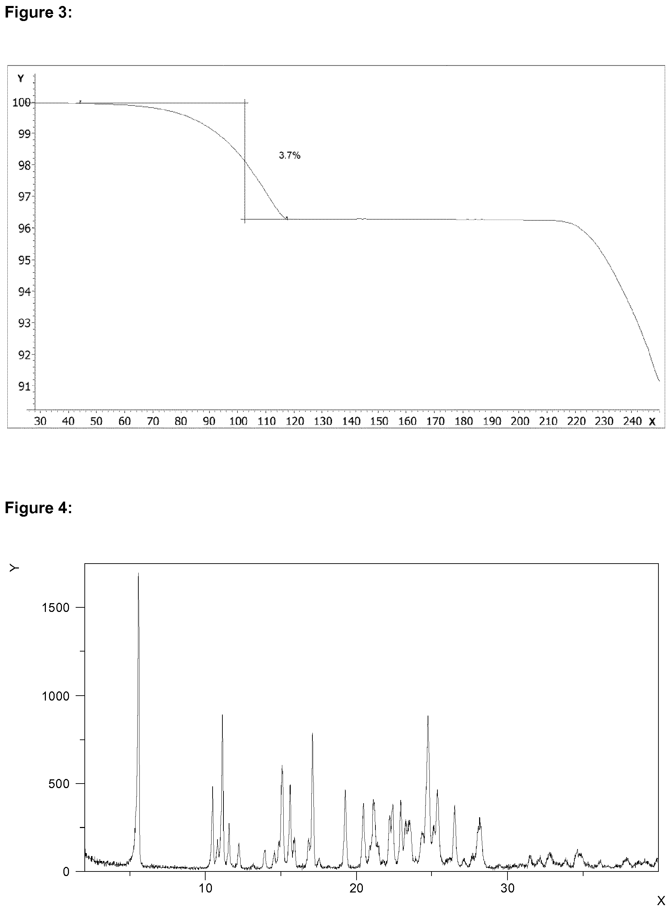

In a first aspect, the present invention relates to crystalline voxelotor napadisylate salts, preferably heminapadisylate salts, in particular crystalline heminapadisylate salt designed as Form A, and a method for preparing the same. In the following, crystalline voxelotor napadisylate salts, in particular Form A, are described:
A-1. Crystalline salt of 2-hydroxy-6-((2-(1-isopropyl-1H-pyrazol-5-yl)pyridin-3-yl)methoxy)benzaldehyde (also referred to as "voxelotor") with 1,5-naphthalenedisulfonic acid.
A-2. Crystalline form according to item A-1, wherein said crystalline form is a heminapadisylate salt, and is preferably Form A, which is characterized by having a powder X-ray diffractogram (PXRD) comprising reflections at 2-Theta angles of (5.0 ± 0.2)°, (11.8 ± 0.2)° and (15.0 ± 0.2), when measured at a temperature in the range of from 20 to 30°C with Cu-Kalpha_{1,2} radiation having a wavelength of 0.15419 nm.
   Form A may be a hydrate, more preferably Form A is a hydrate containing about 1.0 mol of water at ambient conditions, i.e. at a temperature in the range of about 20 to 30 °C and a relative humidity in the range of about 20 to 50%.
A-3. Crystalline form of voxelotor according to item A-1 or A-2, further characterized
   (i) by having a powder X-ray diffractogram comprising reflections at 2-Theta angles of: (5.0 ± 0.2)°, (10.8 ± 0.2)°, (11.8 ± 0.2)° and (15.0 ± 0.2)°, when measured at a temperature in the range of from 20 to 30°C with Cu-Kalpha_{1,2} radiation having a wavelength of 0.15419 nm; or
   (ii) by having a powder X-ray diffractogram comprising reflections at 2-Theta angles of: (5.0 ± 0.2)°, (10.8 ± 0.2)°, (11.8 ± 0.2)°, (12.1 ± 0.2)° and (15.0 ± 0.2)°, when measured at a temperature in the range of from 20 to 30°C with Cu-Kalpha_{1,2} radiation having a wavelength of 0.15419 nm; or
   (iii) by having a powder X-ray diffractogram comprising reflections at 2-Theta angles of: (5.0 ± 0.2)°, (10.8 ± 0.2)°, (11.8 ± 0.2)°, (12.1 ± 0.2)°, (14.5 ± 0.2)° and (15.0 ± 0.2)°, when measured at a temperature in the range of from 20 to 30°C with Cu-Kalpha_{1,2} radiation having a wavelength of 0.15419 nm; or
   (iv) by having a powder X-ray diffractogram comprising reflections at 2-Theta angles of: (5.0 ± 0.2)°, (10.8 ± 0.2)°, (11.8 ± 0.2)°, (12.1 ± 0.2)°, (14.5 ± 0.2)°, (15.0 ± 0.2)° and (15.6 ± 0.2)°, when measured at a temperature in the range of from 20 to 30°C with Cu-Kalpha_{1,2} radiation having a wavelength of 0.15419 nm; or
   (v) by having a powder X-ray diffractogram comprising reflections at 2-Theta angles of: (5.0 ± 0.2)°, (10.8 ± 0.2)°, (11.8 ± 0.2)°, (12.1 ± 0.2)°, (14.5 ± 0.2)°, (15.0 ± 0.2)°, (15.6 ± 0.2)°and (17.1 ± 0.2)°, when measured at a temperature in the range of from 20 to 30°C with Cu-Kalpha_{1,2} radiation having a wavelength of 0.15419 nm; or
   (vi) by having a powder X-ray diffractogram comprising reflections at 2-Theta angles of: (5.0 ± 0.2)°, (10.8 ± 0.2)°, (11.8 ± 0.2)°, (12.1 ± 0.2)°, (14.5 ± 0.2)°, (15.0 ± 0.2)°, (15.6 ± 0.2)°, (17.1 ± 0.2)° and (18.2 ± 0.2)°, when measured at a temperature in the range of from 20 to 30°C with Cu-Kalpha_{1,2} radiation having a wavelength of 0.15419 nm; or
   (vii) by having a powder X-ray diffractogram comprising reflections at 2-Theta angles of: (5.0 ± 0.2)°, (10.8 ± 0.2)°, (11.8 ± 0.2)°, (12.1 ± 0.2)°, (14.5 ± 0.2)°, (15.0 ± 0.2)°, (15.6 ± 0.2)°, (17.1 ± 0.2)°, (18.2 ± 0.2)° and (19.0 ± 0.2)°, when measured at a temperature in the range of from 20 to 30°C with Cu-Kalpha_{1,2} radiation having a wavelength of 0.15419 nm.
A-4. Crystalline form of voxelotor heminapadisylate according to any of items A-2 to A-3, wherein the peaks are not defined by an accuracy of ± 0.2 but by an accuracy of ± 0.1.
A-5. Crystalline form of voxelotor heminapadisylate according to any of items A-2 to A-4, characterized by having a powder X-ray diffractogram essentially the same as shown in Figure 1 herein, when measured at a temperature in the range of from 20 to 30°C with Cu-Kalpha_{1,2} radiation having a wavelength of 0.15419 nm.
A-6. Crystalline form of voxelotor napadisylate according to any of items A-1 to A-5, characterized by a first broad endothermic event between about 80 and 125°C, corresponding to reversible dehydration process, and a second endotherm with an onset temperature of 217.6±1.0°C and a peak maximum at about 218.3±1.0°C, when determined by differential scanning calorimetry (DSC).
   The DSC can be measured at a heating rate of 10°C/min. A representative DSC curve of crystalline voxelotor heminapadisylate Form A is displayed in Figure 2.
A-7. Crystalline form of voxelotor napadisylate according to any of items A-1 to A-6, characterized by having a DSC curve essentially the same as displayed in Figure 2.
A-8. Crystalline form of voxelotor napadisylate according to any of items A-1 to A-7, characterized by a weight loss of about 3.7 wt.% from about 60 to 120°C, corresponding to reversible sample dehydration and loss of eventual unbound residual organic solvent (1.0 mole equivalent of water corresponds to about 3.6 wt.%), when carrying out thermogravimetric analysis (TGA).
A representative TGA curve of crystalline voxelotor heminapadisylate Form A, is provided in Figure 3 herein.
A-9. Crystalline form of voxelotor napadisylate according to any of items A-1 to A-8, characterized by comprising about ≤ 1.0 wt.%, preferably about ≤ 0.8 wt.%, more preferably about ≤ 0.6 wt.% and most preferably about ≤ 0.4 wt.% organic solvent(s).
   The organic solvent can be determined e.g. by TGA and thermogravimetric mass spectroscopy (TGMS).
A-10. Crystalline form of voxelotor napadisylate according to any of items A-1 to A-9, characterized by a water content of 3.3 ± 0.1 wt.% at about 20% RH and a water content of 3.6 ± 0.1 wt.% at about 85% RH, when measured at 25 ± 0.1°C by gravimetric moisture sorption/ desorption (GMSD).

Alternatively or additionally, crystalline form of voxelotor napadisylate, preferably crystalline Form A of voxelotor heminapadisylate, can be characterized by comprising from about 3.3 ± 0.1 to 3.6 ± 0.1 wt.% water in the range of from 20 to 85% relative humidity and at 25 ± 0.1°C. Crystalline Form A can also be characterized by having a water uptake lower than 1.0 wt.%, preferably lower than 0.6 wt.%, even more preferably from 0.1 to 0.5 wt.% in the range of from 20 to 85% relative humidity and 25 ± 0.1°C.

According to the GMSD isotherms, crystalline Form A is a hydrate with low hygroscopicity between 20 and 85% RH. Hence, alternatively or additionally, crystalline Form A can be characterized as being a hydrate.

In particular, crystalline Form A can be a monohydrate.
A-11. Crystalline form of voxelotor napadisylate according to any of items A-1 to A-10, characterized in that it is stable under normal conditions for at least two years, in particular that it does not disproportionate to voxelotor free base.
   Alternatively or additionally, crystalline voxelotor heminapadisylate Form A, can be characterized by being stable upon storage under normal conditions, preferably under storage at a relative humidity of 45% at 23°C for at least two six months, more preferably for at least one year, most preferably for at least two years. Alternatively or additionally, crystalline voxelotor heminapadisylate Form A can be characterized by being stable upon storage under accelerated conditions, preferably under storage at a relative humidity of 70% at 40°C for at least one month, more preferably for at least three months, most preferably for at least six months.
A-12. Crystalline form of voxelotor napadisylate according to any one of items A-1 to A-11, which is in substantially pure form, preferably has a purity of at least 95 wt.%, further preferred at least 98 wt.%.
   The (chemical) purity as referred to herein can be determined by HPLC -analysis.
A-13. Crystalline form of voxelotor napadisylate according to any one of items A-1 to A-12, which is in substantially pure polymorphic form, preferably has a polymorphic purity of at least 95 wt.%, further preferred at least 98 wt.%.
   The polymorphic purity as referred to herein can be determined by PXRD -analysis.
A-14. Crystalline form of voxelotor napadisylate according to any one of items A-1 to A-13, preferably crystalline Form A of voxelotor heminapadisylate, which has a chemical purity of at least 95 wt.%, preferably at least 98%, as determined by HPLC-analysis, and a polymorphic purity of at least 95 wt.%, further preferred at least 98 wt.%, as determined by PXRD analysis.
   The "impurities" are other compounds than voxelotor and 1,5-naphthalenedisulfonic acid as well as other crystalline or non-crystalline forms of voxelotor napadisylate salt.

### Preparation of voxelotor napadisylate salts, in particular Form A:

The present invention also refers to a process for the preparation of crystalline salts of voxelotor napadisylate, preferably voxelotor heminapadisylates, in particular crystalline Form A of voxelotor heminapadisylate. In the following, preferred embodiments of the preparation are described:
A-15. Process for the preparation of crystalline salts of voxelotor napadisylate as defined in any one of items A-1 to A-14, in particular crystalline Form A of voxelotor heminapadisylate, comprising the following steps:
(i) providing voxelotor;
(ii) adding 1,5-naphthalenedisulfonic acid and at least one organic solvent to obtain a solution or a suspension, wherein 1,5-naphthalenedisulfonic acid and/or the at least one organic solvent contain(s) water;
(iii) optionally seeding the mixture with the crystalline form of voxelotor napadisylate;
(iv) subjecting the mixture provided in (iii) to crystallization conditions leading to the formation of voxelotor napadisylate salt;
(v) optionally isolating the obtained crystalline salt of voxelotor;
(vi) optionally drying the obtained crystalline salt of voxelotor;
(vii) optionally equilibrating the obtained crystalline salt of voxelotor with an atmosphere having a relative humidity of from 25% to 80% at a temperature of from 25°C to 40°C;
(viii) thereby obtaining the crystalline salts of voxelotor napadisylate, in particular crystalline Form A of voxelotor heminapadisylate, as defined in any one of items A-1 to A-14.

In step (i) voxelotor may be provided by any method known to the person skilled in the art, such as, e.g., the methods described in WO2015/120133. Furthermore, voxelotor may be provided in any form, such as in crystalline form, in amorphous form, or as a mixture thereof. Voxelotor may be present in crystalline form, e.g. in Form I or Form II or as Material N or as a mixture of two or more thereof. Voxelotor may also be provided as a crystalline solvate reported in WO2015/120133.
In step (ii) 1,5-naphthalenedisulfonic acid - or a hydrated form of 1,5-naphthalenedisulfonic acid - and the at least one organic solvent can be added in any order. Moreover, 1,5-naphthalenedisulfonic acid can be added neat or can be first dissolved or suspended in the at least one organic solvent. In step (ii), the molar ratio of voxelotor and 1,5-naphthalenedisulfonic acid is preferably in the range of from 4.0:1.0 to 1.0:4.0, more preferably in the range of from 2.0:1.0 to 1.0:2.0. In step (ii), the solvent or a solution containing the acid dissolved or suspended in the solvent is added at a temperature in the range of from 15 to 70°C, more preferably in the range of from 20 to 50°C, preferably at ambient pressure. The mixture may optionally be sonicated at a temperature in the range of from 20 to 50 °C to accelerate (partial) dissolution of the solid material.
A suitable organic solvent, in which the acid base reaction of step (ii) may be carried out is selected from the group consisting of acetonitrile, C3-C5 ketones, C1-C2 halogenated hydrocarbons, C2-C4 alcohols, C2-C6 ethers, C3-C5 esters or a combination of two or more thereof. Preferably, the solvent is selected from the group consisting of dichloromethane, chloroform, tetrahydrofuran, 2-methyltetrahydrofuran, ethanol, 2-propanol, ethyl acetate, isopropyl acetate, acetone, acetonitrile or mixtures of two or more thereof.
Preferably, the solvent and 1,5-naphthalenedisulfonic acid are non-anhydrous and contain water in an amount sufficient to allow crystalline Form A of voxelotor heminapadisylate of the present invention to form. The amount of water necessary for the formation of Form A of voxelotor heminapadisylate, which is a hydrate, is low, and usually hydrated form of 1,5-naphthalenedisulfonic acid and bottle of laboratory solvent, which has been open for some time, will contain sufficient amounts. However, ifa nhydrous 1,5-naphthalenedisulfonic acid and anhydrous solvent are used for the process, for example for the production of crystalline Form A of voxelotor heminapadisylate at a larger scale, such as the production of kg quantities, a low amount of water, such as 0.1 wt.%, can be added in order to facilitate formation of Form A of voxelotor heminapadisylate.
Optionally, seed crystals may be added in step (iii). The seed crystals are prepared according to the same process as the one reported in A-15. In particular, the seed crystals are prepared using the same process steps as the specific crystalline Form A of voxelotor heminapadisylate of the present invention. The seed crystals are typically added in an amount of 0.1 wt.% to 10 wt.%, preferably in an amount of 0.5 wt.% to 7.0 wt.%, most preferably 1.0 wt.% 5.0 wt.%, on the basis of the total amount of the starting material used in step (i).
In step (iv) the mixture is subjected to crystallization conditions at a temperature in the range of from 15 to 70°C, preferably in the range from 20 to 50°C, typically for a period of time from 2 to 24 hours, under stirring conditions. After crystallization, the mixture can optionally be cooled to a temperature in the range from 0 to 20°C, preferably in the range from 5 to 15°C and/or one or more anti-solvents can be added to the mixture to increase the process yield. Regarding the anti-solvents, no specific restrictions exist, provided that the crystalline form of voxelotor napadisylate is not soluble and is stable.

Optionally, isolation in step (v) may be performed by using procedures known in the art, such as by filtration, centrifugation, or evaporation of solvent. Moreover, the isolated crystals may optionally be dried in step (vi), e.g. under reduced pressure, typically at room temperature, or heated up to a temperature between 25°C and 40°C and a pressure of below 50 mbar. Overdrying in step (vi) or lack of drying leading to the presence of residual bounded or non-bounded solvent may result in a partial loss of water from crystal Form A or the presence of solvated form of voxelotor heminapadisylate, such as Form C of the present invention. Such a sample can be exposed in step (vii), for example for a period of more than 0.5 hour, such as for more than 6 hours, such as for a period of from 0.5 to 70 hours to an atmosphere having a relative humidity of from 25% to 80% and a temperature of from 25°C to 40°C in order to improve polymorphic purity of the obtained voxelotor heminapadisylate crystal Form A and/or to obtain pure voxelotor heminapadisylate crystal Form A (in step (viii)).
A-16. The present invention relates also to an alternative process for the preparation of crystalline salts of voxelotor napadisylate as defined in any one of items A-1 to A-14, in particular crystalline Form A of voxelotor heminapadisylate, comprising the following steps:
   (i) providing crystalline Form B or Form C of voxelotor heminapadisylate as defined herein;
   (ii) exposing the crystals to an atmosphere having a relative humidity of from 25% to 80% at a temperature of from 25°C to 40°C, for example for a period of more than 0.5 hour, such as for more than 6 hours, such as for a period of from 0.5 to 70 hours;
   (iii) thereby obtaining the crystalline salts of voxelotor napadisylate, in particular crystalline Form A of voxelotor heminapadisylate, as defined in any one of items A-1 to A-14
A-17. The present invention relates to a further alternative process for the preparation of crystalline salts of voxelotor napadisylate as defined in any one of items A-1 to A-14, in particular crystalline Form A of voxelotor heminapadisylate, comprising the following steps:
   (i) providing crystalline Form B and/or Form C of voxelotor heminapadisylate as defined herein;
   (ii) adding water to obtain a suspension;
   (iii) subjecting the mixture provided in (ii) to stirring conditions at a temperature of 20 to 80°C for a period of time of 0.1 to 3 hours;
   (iv) optionally isolating the obtained crystalline salt of voxelotor;
   (v) optionally drying the obtained crystalline salt of voxelotor;
   (vi) thereby obtaining the crystalline salts of voxelotor napadisylate, in particular crystalline Form A of voxelotor heminapadisylate, as defined in any one of items A-1 to A-14.

Steps (iv) and (v) of A-17 correspond to steps (v) and (vi) of A-15. Thus, steps (iv) and (v) of A-17 can be carried out as described above in steps (v) and (vi) of A-15.

### Voxelotor heminapadisylate Form B

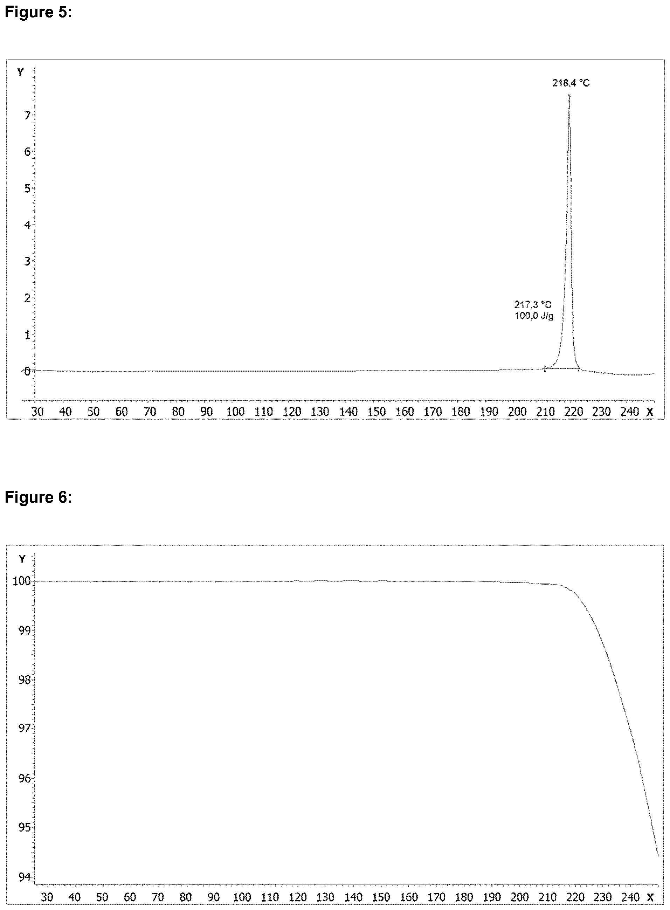

In a second aspect, the present invention relates to a crystalline voxelotor napadisylate salt according to A-1 or B-1, preferably heminapadisylate salts, in particular crystalline heminapadisylate salt designated as Form B, and a method for preparing the same. In the following, preferred embodiments are described:
B-1. Crystalline salt of 2-hydroxy-6-((2-(1-isopropyl-1H-pyrazol-5-yl)pyridin-3-yl)methoxy)benzaldehyde (also referred to as "voxelotor") with 1,5-naphthalenedisulfonic acid.
B-2. Crystalline form according to item B-1, wherein said crystalline form is a heminapadisylate salt, and is preferably Form B, which is characterized by having a powder X-ray diffractogram (PXRD) comprising reflections at 2-Theta angles of (5.6 ± 0.2)°, (10.5 ± 0.2)° and (11.1 ± 0.2), when measured at a temperature in the range of from 20 to 30°C with Cu-Kalpha_{1,2} radiation having a wavelength of 0.15419 nm.
   Form B may be anhydrous or a hydrated form of voxelotor heminapadisylate salt, containing from 0.0 to 0.5 mol of water. Preferably, Form B is a partially or fully dehydrated crystalline form containing from 0.0 to 0.5 mol, preferably from 0.0 to 0.3 mol, more preferably from 0.0 to 0.1 mol of bounded and/or unbounded water. Even more preferably, Form B is an anhydrous form.
B-3. Crystalline form of voxelotor according to item B-1 or B-2, further characterized
   (i) by having a powder X-ray diffractogram comprising reflections at 2-Theta angles of: (5.6 ± 0.2)°, (10.5 ± 0.2)°, (11.1 ± 0.2) and (11.6 ± 0.2)°, when measured at a temperature in the range of from 20 to 30°C with Cu-Kalpha_{1,2} radiation having a wavelength of 0.15419 nm; or
   (ii) by having a powder X-ray diffractogram comprising reflections at 2-Theta angles of: (5.6 ± 0.2)°, (10.5 ± 0.2)°, (11.1 ± 0.2), (11.6 ± 0.2)° and (15.1 ± 0.2)°, when measured at a temperature in the range of from 20 to 30°C with Cu-Kalpha_{1,2} radiation having a wavelength of 0.15419 nm; or
   (iii) by having a powder X-ray diffractogram comprising reflections at 2-Theta angles of: (5.6 ± 0.2)°, (10.5 ± 0.2)°, (11.1 ± 0.2), (11.6 ± 0.2)°, (15.1 ± 0.2)° and (15.6 ± 0.2)°, when measured at a temperature in the range of from 20 to 30°C with Cu-Kalpha_{1,2} radiation having a wavelength of 0.15419 nm; or
   (iv) by having a powder X-ray diffractogram comprising reflections at 2-Theta angles of: (5.6 ± 0.2)°, (10.5 ± 0.2)°, (11.1 ± 0.2), (11.6 ± 0.2)°, (15.1 ± 0.2)°, (15.6 ± 0.2)° and (17.1 ± 0.2)°, when measured at a temperature in the range of from 20 to 30°C with Cu-Kalpha_{1,2} radiation having a wavelength of 0.15419 nm; or
   (v) by having a powder X-ray diffractogram comprising reflections at 2-Theta angles of: (5.6 ± 0.2)°, (10.5 ± 0.2)°, (11.1 ± 0.2), (11.6 ± 0.2)°, (15.1 ± 0.2)°, (15.6 ± 0.2)°, (17.1 ±0.2)° and (19.3 ± 0.2)°, when measured at a temperature in the range of from 20 to 30°C with Cu-Kalpha_{1,2} radiation having a wavelength of 0.15419 nm; or
   (vi) by having a powder X-ray diffractogram comprising reflections at 2-Theta angles of: (5.6 ± 0.2)°, (10.5 ± 0.2)°, (11.1 ± 0.2), (11.6 ± 0.2)°, (15.1 ± 0.2)°, (15.6 ± 0.2)°, (17.1 ± 0.2)°, (19.3 ± 0.2)° and (20.5 ± 0.2)°, when measured at a temperature in the range of from 20 to 30°C with Cu-Kalpha_{1,2} radiation having a wavelength of 0.15419 nm; or
   (vii) by having a powder X-ray diffractogram comprising reflections at 2-Theta angles of: (5.6 ± 0.2)°, (10.5 ± 0.2)°, (11.1 ± 0.2), (11.6 ± 0.2)°, (15.1 ± 0.2)°, (15.6 ± 0.2)°, (17.1 ± 0.2)°, (19.3 ± 0.2)°, (20.5 ± 0.2)° and (21.1 ± 0.2)°, when measured at a temperature in the range of from 20 to 30°C with Cu-Kalpha_{1,2} radiation having a wavelength of 0.15419 nm.
B-4. Crystalline form of hemivoxelotor napadisylate according to any of items B-2 to B-3, wherein the peaks are not defined by an accuracy of ± 0.2 but by an accuracy of ± 0.1.
B-5. Crystalline form of voxelotor heminapadisylate according to any of items B-2 to B-4, characterized by having a powder X-ray diffractogram essentially the same as shown in Figure 4 herein, when measured at a temperature in the range of from 20 to 30°C with Cu-Kalpha_{1,2} radiation having a wavelength of 0.15419 nm.
B-6. Crystalline form of voxelotor napadisylate according to any of items B-1 to B-5, characterized by an endotherm with an onset temperature of 217.3±1.0°C and a peak maximum at about 218.4±1.0°C, when determined by differential scanning calorimetry (DSC). The DSC can be measured at a heating rate of 10°C/min. A representative DSC curve of crystalline voxelotor heminapadisylate Form B is displayed in Figure 5.
B-7. Crystalline form of voxelotor napadisylate according to any of items B-1 to B-6, characterized by having a DSC curve essentially the same as displayed in Figure 5.
B-8. Crystalline form of voxelotor napadisylate according to any of items B-1 to B-7, characterized by no significant weight loss from 25 to about 175°C when carrying out thermogravimetric analysis (TGA).
   A representative TGA curve of crystalline voxelotor heminapadisylate Form B, is provided in Figure 6 herein.
B-9. Crystalline form of voxelotor napadisylate according to any of items B-1 to B-8, characterized by comprising about ≤ 1.0 wt.%, preferably about ≤ 0.8 wt.%, more preferably about ≤ 0.6 wt.% and most preferably about ≤ 0.4 wt.% organic solvent.
   The organic solvent can be determined e.g. by TGA and thermogravimetric mass spectroscopy (TGMS).
B-10. Crystalline form of voxelotor napadisylate according to any one of items B-1 to B-9, which is in substantially pure form, preferably has a purity of at least 95 wt.%, further preferred at least 98 wt.%.
   The (chemical) purity as referred to herein can be determined by HPLC -analysis.
B-11. Crystalline form of voxelotor napadisylate according to any one of items B-1 to B-10, which is in substantially pure polymorphic form, preferably has a polymorphic purity of at least 95 wt.%, further preferred at least 98 wt.%.
   The polymorphic purity as referred to herein can be determined by PXRD -analysis.
B-12. Crystalline form of voxelotor napadisylate according to any one of items B-1 to B-11B, preferably crystalline Form B of voxelotor heminapadisylate, which has a chemical purity of at least 95 wt.%, preferably at least 98%, as determined by HPLC-analysis, and a polymorphic purity of at least 95 wt.%, further preferred at least 98 wt.%, as determined by PXRD analysis.

The "impurities" are other compounds than voxelotor and 1,5-naphthalenedisulfonic acid as well as other crystalline or non-crystalline forms of voxelotor napadisylate salt.

Crystalline Form B of voxelotor heminapadisylate can be used for the preparation of crystalline Form A of voxelotor heminapadisylate of the present invention, for example upon storage at a temperature of about 20 to 40°C and a relative humidity of about 25 to 80 %, such as a temperature of about 40°C and a relative humidity of 75%.

### Preparation of voxelotor heminapadisylate Form B:

The present invention also refers to a process for the preparation of crystalline salts of voxelotor napadisylate, preferably voxelotor heminapadisylates, in particular crystalline Form B of voxelotor heminapadisylate. In the following, preferred embodiments of the preparation are described:
B-13. Process for the preparation of crystalline salts of voxelotor napadisylate as defined in any one of items B-1 to B-12, in particular crystalline Form B of voxelotor heminapadisylate, comprising the following steps:
(i) providing crystalline solvate and/or hydrate of voxelotor heminapadisylate, such as Form A and/or Form C as defined herein; and
(ii) heating and/or exposing the crystal to a temperature in the range of from about 120 to 180°C;
(iii) thereby obtaining the crystalline salts of voxelotor napadisylate, in particular crystalline Form B of voxelotor heminapadisylate, as defined in any one of items B-1 to B-12.

Step (ii) may be performed at atmospheric pressure or at a reduced pressure, for example at a pressure in the range of from 10 mbar to 1000 mbar.

### Voxelotor heminapadisylate Form C

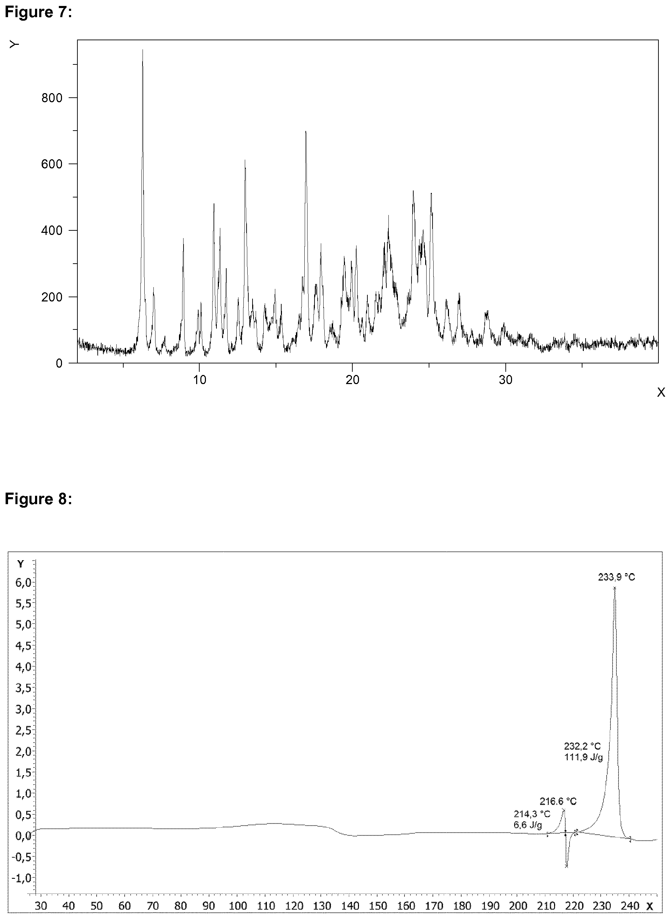

In a further aspect, the present invention relates to crystalline voxelotor napadisylate salts according to A-1 or B-1 or C-1, preferably heminapadisylate salts, in particular crystalline heminapadisylate salt designated as Form C, and a method for preparing the same. In the following, preferred embodiments of Form C are described:
C-1. Crystalline salt of 2-hydroxy-6-((2-(1-isopropyl-1H-pyrazol-5-yl)pyridin-3-yl)methoxy)benzaldehyde (also referred to as "voxelotor") with 1,5-naphthalenedisulfonic acid.
C-2. Crystalline form according to item C-1, which is a heminapadisylate salt, and is preferably Form C, which characterized by having a powder X-ray diffractogram (PXRD) comprising reflections at 2-Theta angles of (6.3 ± 0.2)°, (7.0 ± 0.2)° and (8.9 ± 0.2), when measured at a temperature in the range of from 20 to 30°C with Cu-Kalpha_{1,2} radiation having a wavelength of 0.15419 nm.
   Form C may contain bounded and non-bounded solvent, more preferably Form C may be a solvate. Additionally, Form C may contain bounded and non-bounded water.
C-3. Crystalline form of voxelotor according to item C-1 or C-2, further characterized
   (i) by having a powder X-ray diffractogram comprising reflections at 2-Theta angles of: (6.3 ± 0.2)°, (7.0 ± 0.2)°, (8.9 ± 0.2) and (10.9 ± 0.2)°, when measured at a temperature in the range of from 20 to 30°C with Cu-Kalpha_{1,2} radiation having a wavelength of 0.15419 nm; or
   (ii) by having a powder X-ray diffractogram comprising reflections at 2-Theta angles of: (6.3 ± 0.2)°, (7.0 ± 0.2)°, (8.9 ± 0.2), (10.9 ± 0.2)° and (11.3 ± 0.2)°, when measured at a temperature in the range of from 20 to 30°C with Cu-Kalpha_{1,2} radiation having a wavelength of 0.15419 nm; or
   (iii) by having a powder X-ray diffractogram comprising reflections at 2-Theta angles of: (6.3 ± 0.2)°, (7.0 ± 0.2)°, (8.9 ± 0.2), (10.9 ± 0.2)°, (11.3 ± 0.2)° and (11.7 ± 0.2)°, when measured at a temperature in the range of from 20 to 30°C with Cu-Kalpha_{1,2} radiation having a wavelength of 0.15419 nm; or
   (iv) by having a powder X-ray diffractogram comprising reflections at 2-Theta angles of: (6.3 ± 0.2)°, (7.0 ± 0.2)°, (8.9 ± 0.2), (10.9 ± 0.2)°, (11.3 ± 0.2)°, (11.7 ± 0.2)° and (13.0 ± 0.2)°, when measured at a temperature in the range of from 20 to 30°C with Cu-Kalpha_{1,2} radiation having a wavelength of 0.15419 nm; or
   (v) by having a powder X-ray diffractogram comprising reflections at 2-Theta angles of: (6.3 ± 0.2)°, (7.0 ± 0.2)°, (8.9 ± 0.2), (10.9 ± 0.2)°, (11.3 ± 0.2)°, (11.7 ± 0.2)°, (13.0 ± 0.2)° and (16.9 ± 0.2)°, when measured at a temperature in the range of from 20 to 30°C with Cu-Kalpha_{1,2} radiation having a wavelength of 0.15419 nm; or
   (vi) by having a powder X-ray diffractogram comprising reflections at 2-Theta angles of: (6.3 ± 0.2)°, (7.0 ± 0.2)°, (8.9 ± 0.2), (10.9 ± 0.2)°, (11.3 ± 0.2)°, (11.7 ± 0.2)°, (13.0 ± 0.2)°, (16.9 ± 0.2)° and (17.9 ± 0.2)°, when measured at a temperature in the range of from 20 to 30°C with Cu-Kalpha_{1,2} radiation having a wavelength of 0.15419 nm; or
   (vii) by having a powder X-ray diffractogram comprising reflections at 2-Theta angles of: (6.3 ± 0.2)°, (7.0 ± 0.2)°, (8.9 ± 0.2), (10.9 ± 0.2)°, (11.3 ± 0.2)°, (11.7 ± 0.2)°, (13.0 ± 0.2)°, (16.9 ± 0.2)°, (17.9 ± 0.2)° and (19.5 ± 0.2)°, when measured at a temperature in the range of from 20 to 30°C with Cu-Kalpha_{1,2} radiation having a wavelength of 0.15419 nm.
C-4. Crystalline form of voxelotor heminapadisylate according to any of items C-2 to C-3, wherein the peaks are not defined by an accuracy of ± 0.2 but by an accuracy of ± 0.1.
C-5. Crystalline form of voxelotor heminapadisylate according to any of items C-2 to C-4, characterized by having a powder X-ray diffractogram essentially the same as shown in Figure 7 herein, when measured at a temperature in the range of from 20 to 30°C with Cu-Kalpha_{1,2} radiation having a wavelength of 0.15419 nm.
C-6. Crystalline form of voxelotor napadisylate according to any of items C-1 to C-5, characterized by a first broad endothermic event between about 90 and 135 °C, corresponding to desolvation and/or loss of bounded or unbounded solvent and/or water, a second endotherm with an onset temperature of 214.3 ± 1.0°C and a peak maximum at about 216.6 ± 1.0°C, followed by an exothermic event and then a third endotherm with an onset temperature of 232.2 ± 1.0°C and a peak maximum at about 233.9 ± 1.0°C, when determined by differential scanning calorimetry (DSC).
   The DSC can be measured at a heating rate of 10°C/min. A representative DSC curve of crystalline voxelotor heminapadisylate Form C is displayed in Figure 8.
C-7. Crystalline form of voxelotor napadisylate according to any of items C-1 to C-6, characterized by having a DSC curve essentially the same as displayed in Figure 8.
C-8. Crystalline form of voxelotor napadisylate according to any of items C-1 to C-7, characterized by a weight loss of about 6.0 wt.% from about 35 to about 150°C, corresponding to sample desolvation and/or loss of bounded or unbounded solvent and/or water, when carrying out thermogravimetric analysis (TGA).

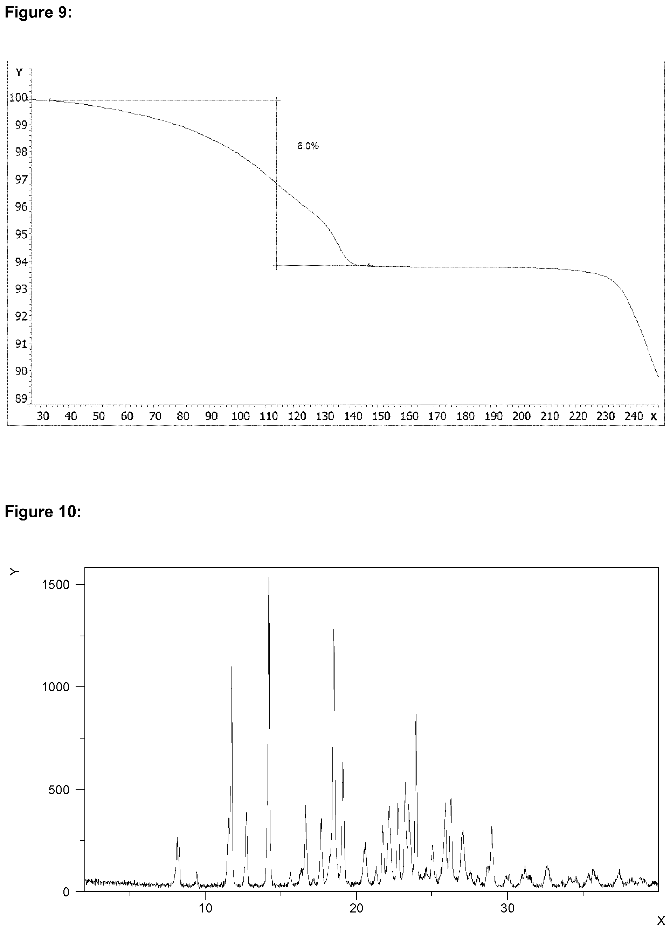

A representative TGA curve of crystalline voxelotor heminapadisylate Form C, is provided in Figure 9 herein.

Crystalline Form C of voxelotor heminapadisylate can be used for the preparation of crystalline Form A, Form B and Form D of voxelotor heminapadisylate of the present invention. For example, crystalline Form A of voxelotor heminapadisylate of the present invention can be prepared by storing crystalline Form C of voxelotor heminapadisylate at a temperature of about 20 to 40°C and a relative humidity of about 25 to 80 %, such as a temperature of about 40°C and a relative humidity of 75% or by suspending crystalline Form C in water at a temperature of about 20 to about 80 °C. Crystalline Form B and Form D of voxelotor heminapadisylate of the present invention can be obtained upon heating and/or storing crystalline Form C to/at a temperature of about 120 to 180°C or a temperature of about 215 to 220°C, respectively.

### Preparation of voxelotor heminapadisylate Form C:

The present invention also refers to a process for the preparation of crystalline salts of voxelotor napadisylate, preferably voxelotor heminapadisylates, in particular crystalline Form C of voxelotor heminapadisylate. In the following, preferred embodiments of the preparation are described:
C-9. Process for the preparation of crystalline salts of voxelotor napadisylate as defined in any one of items C-1 to C-8, in particular crystalline Form C of voxelotor heminapadisylate, comprising the following steps:
(i) providing voxelotor;
(ii) adding 1,5-naphthalenedisulfonic acid and at least one organic solvent to obtain a solution or a suspension;
(iii) optionally seeding the mixture with the crystalline form of voxelotor heminapadisylate;
(iv) subjecting the mixture provided in (iii) to crystallization conditions leading to the formation of the crystalline form of voxelotor heminapadisylate;
(v) optionally isolating the obtained crystalline salt of voxelotor;
(vi) optionally drying the obtained crystalline salt of voxelotor;
(vii) thereby obtaining the crystalline salts of voxelotor napadisylate, in particular crystalline Form C of voxelotor heminapadisylate, as defined in any one of items C-1 to C-8.

In step (i) voxelotor may be provided by any method known to the person skilled in the art, such as, e.g., the methods described in WO2015/120133. Furthermore, voxelotor may be provided in any form, such as in crystalline form, in amorphous form, or as a mixture thereof. Voxelotor may be present in crystalline form, e.g. in Form I or Form II or as Material N or as a mixture of two or more thereof. Voxelotor may also be provided as a crystalline solvate reported in WO2015/120133.
In step (ii) 1,5-naphthalenedisulfonic acid - or a hydrated form of 1,5-naphthalenedisulfonic acid - and the at least one organic solvent can be added in any order. Moreover, 1,5-naphthalenedisulfonic acid can be added neat or can be first dissolved or suspended in the at least one organic solvent. In step (ii), the mole ratio of voxelotor and 1,5-naphthalenedisulfonic acid is preferably in the range of from 4.0:1.0 to 1.0:4.0, more preferably in the range of from 2.0:1.0 to 1.0:2.0. In step (ii), the solvent or a solution containing the acid dissolved or suspended in the solvent is added at a temperature in the range of from 15 to 70°C, more preferably in the range of from 20 to 60°C, preferably at ambient pressure. The mixture may optionally be sonicated at a temperature in the range of from 20 to 50 °C to accelerate (partial) dissolution of the solid material.
A suitable organic solvent, in which the acid base reaction of step (ii) may be carried out is selected from the group consisting of acetonitrile, C3-C5 ketones, C1-C2 halogenated hydrocarbons, C2-C4 alcohols, C2-C6 ethers, C3-C5 esters or a combination of two or more thereof. Preferably, the solvent is selected from the group consisting of dichloromethane, chloroform, tetrahydrofuran, 2-methyltetrahydrofuran, ethanol, 2-propanol, ethyl acetate, isopropyl acetate, acetone, acetonitrile or mixtures of two or more thereof. More preferably, the solvent is ethanol.
Optionally, seed crystals may be added in step (iii). The seed crystals are prepared according to the same process as the one reported in C-9. In particular, the seed crystals are prepared using the same process steps as the specific crystalline Form C of voxelotor heminapadisylate of the present invention. The seed crystals are typically added in an amount of 0.1 wt.% to 10 wt.%, preferably in an amount of 0.5 wt.% to 7.0 wt.%, most preferably 1.0 wt.% 5.0 wt.%, on the basis of the total amount of the starting material used in step (i).
In step (iv) the mixture is subjected to crystallization conditions at a temperature in the range of from 20 to 70°C, preferably in the range from 20 to 60°C, typically for a period of time from 2 to 24 hours, under stirring conditions. After crystallization, the mixture can optionally be cooled to a temperature in the range from 0 to 20°C, preferably in the range from 5 to 15°C and/or one or more anti-solvents can be added to the mixture to increase the process yield. Regarding the anti-solvents, no specific restrictions exist, provided that the crystalline form of voxelotor heminapadisylate is not soluble and is stable.
Optionally, isolation in step (v) may be performed by using procedures known in the art, such as by filtration, centrifugation, or evaporation of solvent. Moreover, the isolated crystals may optionally be dried in step (vi), e.g. under reduced pressure, typically at room temperature and a pressure of below 50 mbar.

### Voxelotor heminapadisylate Form D:

In another aspect, the present invention relates to crystalline voxelotor napadisylate salt according to A-1 or B-1 or C-1 or D-1, preferably heminapadisylate salts, in particular crystalline heminapadisylate salt designated as Form D, and a method for preparing the same. In the following, preferred embodiments of Form D are described:
D-1. Crystalline salt of 2-hydroxy-6-((2-(1-isopropyl-1H-pyrazol-5-yl)pyridin-3-yl)methoxy)benzaldehyde (also referred to as "voxelotor") with 1,5-naphthalenedisulfonic acid.
D-2. Crystalline form according to item D-1, wherein said crystalline form is a heminapadisylate salt, and is preferably Form D, which is characterized by having a powder X-ray diffractogram (PXRD) comprising reflections at 2-Theta angles of (8.1 ± 0.2)°, (11.7 ± 0.2)° and (14.2 ± 0.2), when measured at a temperature in the range of from 20 to 30°C with Cu-Kalpha_{1,2} radiation having a wavelength of 0.15419 nm.
   Form D may be anhydrous or a hydrated form of voxelotor heminapadisylate salt, containing from 0.0 to 0.5 mol of water. Preferably, Form D is an anhydrous form.
D-3. Crystalline form of voxelotor according to item D-1 or D-2, further characterized
   (i) by having a powder X-ray diffractogram comprising reflections at 2-Theta angles of: (8.1 ± 0.2)°, (11.7 ± 0.2)°, (12.7 ± 0.2)° and (14.2 ± 0.2), when measured at a temperature in the range of from 20 to 30°C with Cu-Kalpha_{1,2} radiation having a wavelength of 0.15419 nm; or
   (ii) by having a powder X-ray diffractogram comprising reflections at 2-Theta angles of: (8.1 ± 0.2)°, (11.7 ± 0.2)°, (12.7 ± 0.2)°, (14.2 ± 0.2), and (16.6 ± 0.2)°, when measured at a temperature in the range of from 20 to 30°C with Cu-Kalpha_{1,2} radiation having a wavelength of 0.15419 nm; or
   (iii) by having a powder X-ray diffractogram comprising reflections at 2-Theta angles of: (8.1 ± 0.2)°, (11.7 ± 0.2)°, (12.7 ± 0.2)°, (14.2 ± 0.2), (16.6 ± 0.2)° and (17.7 ± 0.2)°, when measured at a temperature in the range of from 20 to 30°C with Cu-Kalpha_{1,2} radiation having a wavelength of 0.15419 nm; or
   (iv) by having a powder X-ray diffractogram comprising reflections at 2-Theta angles of: (8.1 ± 0.2)°, (11.7 ± 0.2)°, (12.7 ± 0.2)°, (14.2 ± 0.2), (16.6 ± 0.2)°, (17.7 ± 0.2)° and (18.5 ± 0.2)°, when measured at a temperature in the range of from 20 to 30°C with Cu-Kalpha_{1,2} radiation having a wavelength of 0.15419 nm; or
   (v) by having a powder X-ray diffractogram comprising reflections at 2-Theta angles of: (8.1 ± 0.2)°, (11.7 ± 0.2)°, (12.7 ± 0.2)°, (14.2 ± 0.2), (16.6 ± 0.2)°, (17.7 ± 0.2)°, (18.5 ± 0.2)° and (19.1 ± 0.2)°, when measured at a temperature in the range of from 20 to 30°C with Cu-Kalpha_{1,2} radiation having a wavelength of 0.15419 nm; or
   (vi) by having a powder X-ray diffractogram comprising reflections at 2-Theta angles of: (8.1 ± 0.2)°, (11.7 ± 0.2)°, (12.7 ± 0.2)°, (14.2 ± 0.2), (16.6 ± 0.2)°, (17.7 ± 0.2)°, (18.5 ± 0.2)°, (19.1 ± 0.2)° and (20.6 ± 0.2)°, when measured at a temperature in the range of from 20 to 30°C with Cu-Kalpha_{1,2} radiation having a wavelength of 0.15419 nm; or
   (vii) by having a powder X-ray diffractogram comprising reflections at 2-Theta angles of: (8.1 ± 0.2)°, (11.7 ± 0.2)°, (12.7 ± 0.2)°, (14.2 ± 0.2), (16.6 ± 0.2)°, (17.7 ± 0.2)°, (18.5 ± 0.2)°, (19.1 ± 0.2)°, (20.6 ± 0.2)° and (21.7 ± 0.2)°, when measured at a temperature in the range of from 20 to 30°C with Cu-Kalpha_{1,2} radiation having a wavelength of 0.15419 nm.
D-4. Crystalline form of voxelotor heminapadisylate according to any of items D-2 to D-3, wherein the peaks are not defined by an accuracy of ± 0.2 but by an accuracy of ± 0.1.
D-5. Crystalline form of voxelotor heminapadisylate according to any of items D-2 to D-4, characterized by having a powder X-ray diffractogram essentially the same as shown in Figure 10 herein, when measured at a temperature in the range of from 20 to 30°C with Cu-Kalpha_{1,2} radiation having a wavelength of 0.15419 nm.
   In one embodiment, the crystalline voxelotor napadisylate according to any of items D-1 to D-5 is characterized by having a melting point between 232-236°C, when determined by applying DSC as disclosed herein. It is noted that in Figure 9, the last endothermic event corresponds to the melting of Form D.
D-6. Crystalline form of voxelotor napadisylate according to any of items D-1 to D-5, characterized by comprising about ≤ 1.0 wt.%, preferably about ≤ 0.8 wt.%, more preferably about ≤ 0.6 wt.% and most preferably about ≤ 0.4 wt.% organic solvent.
   The organic solvent can be determined e.g., by TGA and thermogravimetric mass spectroscopy (TGMS).
D-7. Crystalline form of voxelotor napadisylate according to any one of items D-1 to D-6, which is in substantially pure form, preferably has a purity of at least 95 wt.%, further preferred at least 98 wt.%.
   The (chemical) purity as referred to herein can be determined by HPLC -analysis.
D-8. Crystalline form of voxelotor napadisylate according to any one of items D-1 to D-7, which is in substantially pure polymorphic form, preferably has a polymorphic purity of at least 95 wt.%, further preferred at least 98 wt.%.
   The polymorphic purity as referred to herein can be determined by PXRD -analysis.
D-9. Crystalline form of voxelotor napadisylate according to any one of items D-1 to D-8, preferably crystalline Form D of voxelotor heminapadisylate, which has a chemical purity of at least 95 wt.%, preferably at least 98%, as determined by HPLC-analysis, and a polymorphic purity of at least 95 wt.%, further preferred at least 98 wt.%, as determined by PXRD analysis.
   The "impurities" are other compounds than voxelotor and 1,5-naphthalenedisulfonic acid as well as other crystalline or non-crystalline forms of voxelotor napadisylate salt.

### Preparation of voxelotor heminapadisylate Form D:

The present invention also refers to a process for the preparation of crystalline salts of voxelotor napadisylate, preferably voxelotor heminapadisylates, in particular crystalline Form D of voxelotor heminapadisylate. In the following, preferred embodiments of the preparation are described:
D-10. Process for the preparation of crystalline salts of voxelotor napadisylate as defined in any one of items D-1 to D-9, in particular crystalline Form D of voxelotor heminapadisylate, comprising the following steps:
(i) providing crystalline voxelotor heminapadisylate, in particular Form C as defined herein; and
(ii) exposing the crystals to an elevated temperature;
(iii) thereby obtaining the crystalline salts of voxelotor napadisylate, in particular Form D of voxelotor heminapadisylate, as defined in any of items D-1 to D-9.

Step (ii) may be performed at atmospheric pressure or at a reduced pressure, for example at a pressure in the range of from 10 mbar to 1000 mbar, in the presence or the absence of a solvent.

### Pharmaceutical compositions and dosage forms comprising the crystalline forms of voxelotor napadisylate/heminapadisylate

In a further aspect, the present invention also refers to pharmaceutical compositions comprising, preferably consisting of, crystalline salts of voxelotor napadisylate, more preferably voxelotor heminapadisylates, in particular crystalline forms of voxelotor heminapadisylate as defined herein, and one or more pharmaceutically acceptable excipients.

Preferred embodiments of pharmaceutical compositions comprise crystalline Form A and/or crystalline Form B and/or crystalline Form D as defined herein.

The present invention also refers to a method for preparing the same.

In the following, preferred embodiments of compositions comprising the crystalline forms of voxelotor napadisylate, more preferably comprising the crystalline forms of voxelotor heminapadisylate as defined herein, are described.
PC-1. Pharmaceutical composition comprising one or more, preferably one, crystalline form(s) of voxelotor napadisylate as described herein, such as crystalline Form A and/or crystalline Form B and/or crystalline Form D as defined herein, preferably Form A as defined in any of items A-1 to A-14, and/or Form D as defined in any of items D-1 to D-9, and one or more pharmaceutically acceptable excipients.
PC-2. Pharmaceutical composition according to item PC-1, wherein the pharmaceutically acceptable excipients are selected from the group consisting of carriers, fillers, diluents, lubricants, sweeteners, stabilizing agents, solubilizing agents, antioxidants and preservatives, flavouring agents, binders, colorants, osmotic agents, buffers, disintegrants, granulating agents, coating materials and combinations thereof.
PC-3. Pharmaceutic composition according to item PC-1 or PC-2, wherein no wetting agent as defined in the "Definition" part is contained in the pharmaceutical composition, preferably, no wetting agent at all is contained.
PC-4. Pharmaceutical composition according to any one of items PC-1 to PC-3, wherein the amount of microcrystalline cellulose that is present in the pharmaceutical composition is in the range of between 1 wt.% to 100 wt.%, preferably in the range of between 5 wt.% to 20 wt.% , based on the total amount of the pharmaceutical composition.
PC-5. Dosage form comprising the pharmaceutical composition as defined in any of PC-1 to PC-4.

Below are two examples of pharmaceutical compositions of the present invention, comprising crystalline Form A of voxelotor heminapadisylate as defined herein:

| Ingredient | Function | Unit weight (mg/tablet) | Unit weight (mg/tablet) |
|---|---|---|---|
| Voxelotor heminapadisylate monohydrate salt (Form A) | Active | 444,2 | 148,1 |
| equivalent to mg free base | | 300,0 | 100,0 |
| Microcrystalline Cellulose | Filler | 38,3 | 12,8 |
| Croscarmellose Sodium | Disintegrant | 6,3 | 2,1 |
| Magnesium Stearate | Lubricant | 11,3 | 3,8 |
| Tablet core weight | | 500,0 | 166,7 |
| Opadry II | Coating | 10,0 | 3,3 |
| Film coated tablet weight | | 510,0 | 170,0 |

### Use of crystalline forms of voxelotor napadisylate and pharmaceutical composition

In a further aspect, the present invention also refers to the use of the crystalline forms of voxelotor napadisylate as defined herein, and of the pharmaceutical composition comprising the crystalline forms of voxelotor napadisylate as defined herein for the preparation of a pharmaceutical dosage form and for use in a medical treatment.

The crystalline forms of voxelotor napadisylate are as defined in any one of items A-1 to A-14, B-1 to B-12, C-1 to C-8, or D-1 to D-9, preferably Form A as defined in any of items A-1 to A-14.

U-1. Use of the crystalline form of voxelotor napadisylate as defined in any of items A-1 to A-14, B-1 to B-12, C-1 to C-8, or D-1 to D-9, or of the pharmaceutical composition as defined in any one of PC-1 to PC-4, for the preparation of a pharmaceutical dosage form.

Preferably, the crystalline form of voxelotor is voxelotor heminapadisylate.

In a preferred embodiment, the dosage form is a solid oral dosage form, preferably the solid oral dosage form is a tablet.

Finally, the present invention also refers to the crystalline forms of voxelotor napadisylate as defined in any one of items A-1 to A-14, B-1 to B-12, C-1 to C-8, or D-1 to D-9, or the pharmaceutical composition as defined in any one of items PC-1 to PC-4, for use in a method of treating disorders mediated by hemoglobin and disorders that would benefit from increased tissue oxygenation. Preferably, the disorder is stickle cell anemia.

### Methods

### Powder X-ray diffraction

Powder X-ray diffraction (PXRD) was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels).

### Differential scanning calorimetry

Differential scanning calorimetry (DSC) was performed on a Mettler Polymer DSC R instrument. The sample was heated in a 40 microL aluminum pan with pierced aluminum lid from 25 to 250°C at a rate of 10°C/min. Nitrogen (purge rate 50 mL/min) was used as purge gas.

### Thermogravimetric analysis (TGA)

Thermogravimetric analysis (TGA) was performed on a Mettler Toledo TGA/DSC 1 instrument. Samples were heated in 100 µl aluminium pans closed with aluminium lids. Lids were automatically pierced at the beginning of the measurement. Samples were initially kept at 25°C for 2.5 minutes and then heated from 25 to 250°C at a rate of 10°C/min. Nitrogen (purge rate 50 mL/min) was used as purge gas.

Mass spectrometry (MS) measurements, coupled with thermogravimetric analysis (TGA) are performed with a Pfeiffer GSD 320 ThermoStar Gas Analysis System. A part of the headspace was evacuated with a capillary placed near the TG sample pan, and mass fragments between 1 - 100 atomic mass units (amu) were analyzed simultaneously without further separation applying a secondary electron multiplier (SEM) for signal amplification. Since samples were kept at 25°C for 2.5 minutes and then heated with a heating rate of 10°C/min, the ordinate of mass trace printouts, displayed in minutes, can be directly related to temperatures applying a factor of 10 (e.g. 10 minutes correspond to 100°C in the TGA experiment).

### Gravimetric moisture sorption/desorption

Gravimetric moisture sorption/desorption (GMSD) isotherms were recorded with an SPSx-1µ moisture sorption analyzer (ProUmid, Ulm). The measurement cycle was started at relative humidity (RH) between 3% and 45%. Relative humidity was then decreased to 5% RH in 5% steps, followed by a further decrease to 3% RH and to 0% RH. For this isotherm, a black filled square with a white plus ("+") inside is used in the respective figures. Afterwards RH was increased from 0% to 85% RH or form 0% to 90% RH in a sorption cycle and decreased to 0 % in a desorption cycle in 5% steps. Finally RH was increased to ambient relative humidity, i.e. a relative humidity in the range of from 25% to 45%, in 5% steps. As to the isotherm obtained by the last step, a black filled square with a white "x" inside is used in the respective figures.

The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1°C.

### Determination of the moisture stability

Moisture stability at accelerated stability conditions was performed in a Memmert constant climate chamber HPP110. 50-70 mg of crystalline sample were exposed to an atmosphere having a relative humidity of 75±1% and a temperature of 40±0.5°C for a certain period of time.

### Drop Shape Analysis

Drop shape analysis (DSA) gives valuable information about the wetting behavior and the surface free energy (SFE) of a material. The shadow images of the sessile drops were captured by Kruess DSA25E (Kruess, D-Hamburg, Germany), equipped with illumination, a manual lift table (z-axis), video camera and software Kruess Advance 1.6.2. Water was used as liquid. Two contact angles were measured. This standard method is used to get polar and disperse interactions on wettability and adhesion.

A well-prepared smooth and flat surface is needed in the DSA. The use of tablet compacts is a good choice, but often the pure API-Powder has insufficient compactibility. A valid mold lubrication procedure was applied - which had no impact on the DSA result. The water droplets were placed on the surface of a flat-faced tablet and the contact angle (CA) of the sessile drop at the moment of the first contact was measured (by video analysis, frame by frame).

A manual laboratory press (PW10, P/O Weber, D-Remshalden) equipped with force and displacement sensors (Hottinger Baldwin, D-Darmstadt) was used to manufacture the tablets.

### Examples

### Example 1: preparation of crystalline Form A of voxelotor heminapadisylate

### Example 1-1

To a solid mixture containing voxelotor (50.1 mg, crystalline mixture of Form I and Material N, e.g. prepared according to the procedure disclosed in WO2015/120133 in Example 8) and 1,5-naphthalenedisulfonic acid tetrahydrate (31.2 mg, 1.05 equivalent) was added 1.0 mL of warm ethanol. The mixture was sonicated at a temperature of about 40°C leading to direct precipitation. The obtained suspension was stirred at room temperature for about 20 hours, followed by filtration. The collected solid material was dried at a temperature of about 40°C under vacuum (about 30 mbar) for 16 hours, yielding crystalline Form A of voxelotor heminapadisylate as a white-off powder. The PXRD pattern of the obtained crystalline Form A of voxelotor heminapadisylate was essentially identical to the one shown in Figure 1.

### Example 1-2

To a solid mixture containing voxelotor (200.6 mg, crystalline mixture of Form I and Material N, e.g. prepared according to the procedure disclosed in WO2015/120133 in Example 8) and 1,5-naphthalenedisulfonic acid tetrahydrate (225.4 mg, 1.05 equivalent) were added 4.0 mL of warm ethanol. The mixture was sonicated at a temperature of about 40°C leading to direct precipitation. The obtained suspension was stirred at room temperature for about 18 hours, followed by filtration. The collected solid material was washed with ethanol and dried at room temperature under vacuum (about 30 mbar) for 15 hours, followed by storage at ambient conditions (about 25 to 30 °C and 40 to 50 % relative humidity for 3 days), yielding 285 mg of crystalline Form A of voxelotor heminapadisylate. Characteristic PXRD pattern of the obtained crystalline Form A of voxelotor heminapadisylate is shown in Figure 1. The corresponding reflection list is provided in Table 1 below (relative peak intensities can vary due to e.g. inter-apparatus variability, sample crystallinity, sample preparation, etc.).

**Table 1: PXRD reflection list of the crystalline Form A of voxelotor heminapadisylate**

| Pos. [°2θ] | Rel. Int. [%] |
|---|---|
| 5.0 | 40 |
| 10.8 | 38 |
| 11.0 | 16 |
| 11.8 | 43 |
| 12.1 | 20 |
| 14.5 | 19 |
| 15.0 | 100 |
| 15.6 | 21 |
| 16.3 | 3 |
| 17.1 | 25 |
| 18.2 | 26 |
| 19.0 | 37 |
| 20.0 | 4 |
| 22.0 | 15 |
| 22.3 | 33 |
| 22.5 | 26 |
| 23.6 | 28 |
| 23.8 | 12 |
| 24.6 | 41 |
| 25.1 | 23 |
| 25.6 | 13 |
| 26.0 | 36 |
| 26.3 | 6 |
| 27.0 | 12 |
| 27.3 | 21 |
| 27.9 | 12 |
| 29.3 | 6 |
| 31.8 | 7 |
| 34.5 | 6 |

### Example 1-3

Crystalline Form C of voxelotor heminapadisylate (700 mg, prepared according to Example 3 herein) was exposed to an atmosphere having a relative humidity of about 75% and a temperature of about 40°C in a Memmert constant climate chamber HPP110 for 65 hours, yielding crystalline Form A of voxelotor heminapadisylate. The PXRD pattern of the obtained crystalline Form A of voxelotor heminapadisylate was essentially identical to the one shown in Figure 1.

### Example 2: Observation and/or preparation of crystalline Form B of voxelotor heminapadisylate

### Example 2-1

Crystalline Form C of voxelotor heminapadisylate (about 20 mg, prepared according to Example 3 herein) was heated from 25 to 180°C at a rate of 10°C/min and further exposed to a temperature of 180°C for 10 minutes, yielding crystalline Form B of voxelotor heminapadisylate, as a mixture of crystalline Form B and crystalline Form D of voxelotor heminapadisylate.

### Example 2-2

Crystalline Form A of voxelotor heminapadisylate (about 30 mg, prepared according Example 1-2 herein) was dehydrated under heating from 25 to 180°C at a rate of 10°C/min and further exposition to a temperature of 180°C for 10 minutes, yielding crystalline Form B of voxelotor heminapadisylate. Characteristic PXRD pattern of the obtained crystalline Form B of voxelotor heminapadisylate is shown in Figure 4. The corresponding reflection list is provided in Table 2 below (relative peak intensities can vary due to e.g. inter-apparatus variability, sample crystallinity, sample preparation, etc.).

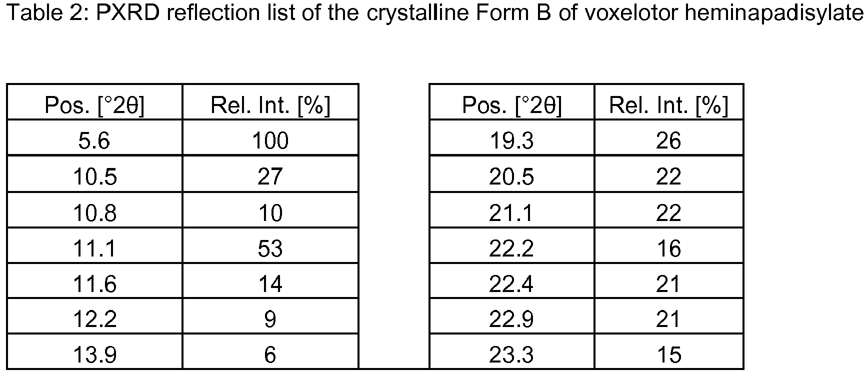

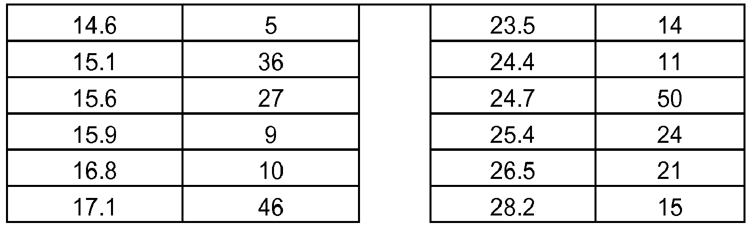

### Example 3: preparation of crystalline Form C of voxelotor heminapadisylate

To a solid mixture containing voxelotor (700.1 mg, crystalline mixture of Form I and Material N, e.g. prepared according to the procedure disclosed in WO2015/120133 in Example 8) and 1,5-naphthalenedisulfonic acid tetrahydrate (793.1 mg, 1.05 equivalent) were added 13.0 mL of warm ethanol, leading to partial dissolution of the solid materials followed by direct solid precipitation under stirring. The obtained suspension was stirred at about 60°C for about 5 minutes and then at room temperature for about 16 hours. Afterwards the suspension was filtered. The collected solid material was washed with ethanol and dried at room temperature under vacuum (about 30 mbar) for 24 hours, yielding 1002.0 mg of crystalline Form C of voxelotor heminapadisylate as white-off powder. Characteristic PXRD pattern of the obtained crystalline Form C of voxelotor heminapadisylate is shown in Figure 7. The corresponding reflection list is provided in Table 3 below (relative peak intensities can vary due to e.g. inter-apparatus variability, sample crystallinity, sample preparation, etc.).

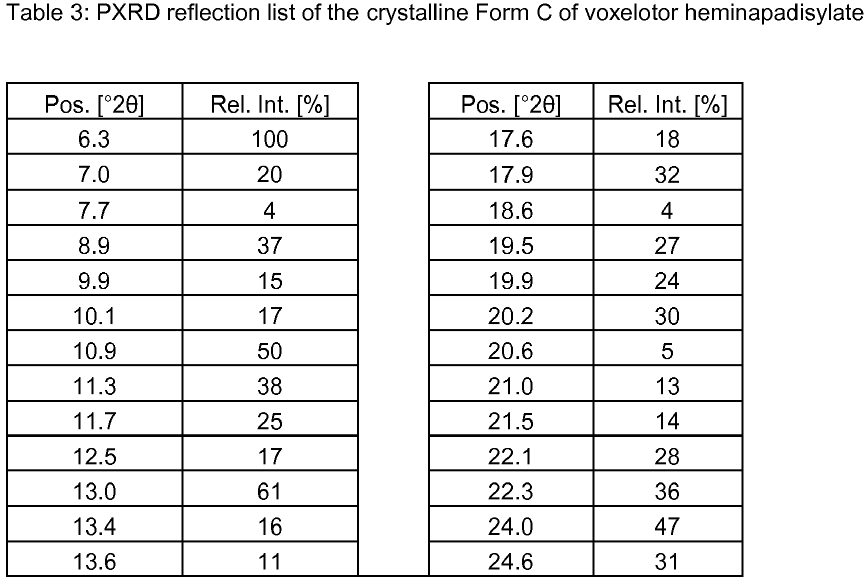

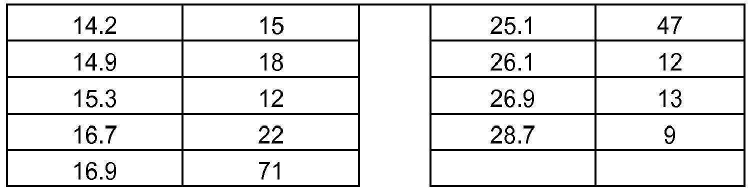

### Example 4: preparation of crystalline Form D of voxelotor heminapadisylate

Crystalline Form C of voxelotor heminapadisylate (about 20 mg, prepared according to Example 3 herein) was heated from 25 to 218°C at a rate of 10°C/min, yielding crystalline Form D of voxelotor heminapadisylate. Characteristic PXRD pattern of the obtained crystalline Form D of voxelotor heminapadisylate is shown in Figure 10. The corresponding reflection list is provided in Table 4 below (relative peak intensities can vary due to e.g. inter-apparatus variability, sample crystallinity, sample preparation, etc.).

**Table 4: PXRD reflection list of the crystalline Form D of voxelotor heminapadisylate**

| Pos. [°2θ] | Rel. Int. [%] |
|---|---|
| 8.1 | 15 |
| 8.3 | 13 |
| 9.4 | 5 |
| 11.5 | 22 |
| 11.7 | 71 |
| 12.7 | 24 |
| 14.2 | 100 |
| 15.6 | 4 |
| 16.6 | 26 |
| 17.7 | 22 |
| 18.5 | 83 |
| 19.1 | 40 |
| 20.6 | 12 |
| 21.3 | 5 |
| 21.7 | 19 |
| 22.2 | 25 |
| 22.8 | 26 |
| 23.2 | 32 |
| 23.5 | 25 |
| 23.9 | 57 |
| 25.1 | 13 |
| 25.9 | 24 |
| 26.2 | 27 |
| 27.0 | 17 |
| 28.9 | 19 |

### Comparative Example 1: comparison of the wetting properties of crystalline Form A of voxelotor heminapadisylate and crystalline Form II of voxelotor free base

### Preparation of voxelotor free base (Form II):

To voxelotor (950-970 mg, crystalline mixture of Form I and Material N, e.g. prepared according to the procedure disclosed in WO2015/120133 in Example 8) were added 10 mL of solvent (DI water or n-heptane). The obtained suspension was stirred at about 60 °C for seven days, followed by filtration. The collected solid material was washed with solvent (DI water or n-heptane) and dried at 25-40°C under vacuum (about 30 mbar) for 18 hours, yielding crystalline Form II of voxelotor.

### Determination of the wetting properties:

The wetting properties of crystalline Form II of voxelotor (free base, prepared according to the process described above) and crystalline Form A of voxelotor heminapadisylate of the present invention (prepared according to Example 1-3 herein) were determined by drop shape analysis. The results are summarized in Table 5.

**Table 5: Results of the drop shape analysis of voxelotor samples (free base and heminapadisylate salt)**

| **Sample** | **Contact angle** | **Comment⁽²⁾** |
|---|---|---|
| Voxelotor free base (Form II) | (98.6 ± 2.1)°⁽¹⁾ | Low wettability / hydrophobic material |
| Voxelotor heminapadisylate (Form A) | (50.3 ± 1.4)° | Good wettability / hydrophilic material |

| | | |
|---|---|---|
| ⁽¹⁾ Average value based on the analysis of two different charges of voxelotor Form II. ⁽²⁾ Interpretation of the CA value according to: Renate Förch; Holger Schonherr; A. Tobias A. Jenkins (2009). Surface design: applications in bioscience and nanotechnology. Wiley-VCH. p. 471. | | |

## Claims

1. Crystalline salt of voxelotor (2-hydroxy-6-((2-(1-isopropyl-1H-pyrazol-5-yl)pyridin-3-yl)methoxy)benzaldehyde) with 1,5-naphthalenedisulfonic acid.

2. Crystalline form according to claim 1, which is a heminapadisylate salt, and is preferably Form A, which is **characterized by** having a powder X-ray diffractogram (PXRD) comprising reflections at 2-Theta angles, when measured at a temperature in the range of from 20 to 30°C with Cu-Kalpha_{1,2} radiation having a wavelength of 0.15419 nm, of:
(i) (5.0 ± 0.2)°, (11.8 ± 0.2)° and (15.0 ± 0.2)°; or
(ii) (5.0 ± 0.2)°, (10.8 ± 0.2)°, (11.8 ± 0.2)° and (15.0 ± 0.2)°; or
(iii) (5.0 ± 0.2)°, (10.8 ± 0.2)°, (11.8 ± 0.2)°, (12.1 ± 0.2)° and (15.0 ± 0.2)°.

3. Crystalline form of voxelotor napadisylate according to claim 1 or 2, **characterized by** a water content of 3.3 ± 0.1 wt.% at about 20% RH and a water content of 3.6 ± 0.1 wt.% at about 85% RH, when measured at 25 ± 0.1°C by gravimetric moisture sorption / desorption (GMSD).

4. Crystalline form according to claim 1, which is a heminapadisylate salt, and is preferably Form B, which is **characterized by** having a powder X-ray diffractogram (PXRD) comprising reflections at 2-Theta angles, when measured at a temperature in the range of from 20 to 30°C with Cu-Kalpha_{1,2} radiation having a wavelength of 0.15419 nm, of
(i) (5.6 ± 0.2)°, (10.5 ± 0.2)° and (11.1 ± 0.2)°; or
(ii) (5.6 ± 0.2)°, (10.5 ± 0.2)°, (11.1 ± 0.2)° and (11.6 ± 0.2)°; or
(ii) (5.6 ± 0.2)°, (10.5 ± 0.2)°, (11.1 ± 0.2), (11.6 ± 0.2)° and (15.1 ± 0.2)°.

5. Crystalline form according to claim 1, which is a heminapadisylate salt, and is preferably Form C, which is **characterized by** having a powder X-ray diffractogram (PXRD) comprising reflections at 2-Theta angles, when measured at a temperature in the range of from 20 to 30°C with Cu-Kalpha_{1,2} radiation having a wavelength of 0.15419 nm, of:
(i) (6.3 ± 0.2)°, (7.0 ± 0.2)° and (8.9 ± 0.2)°; or
(ii) (6.3 ± 0.2)°, (7.0 ± 0.2)°, (8.9 ± 0.2) and (10.9 ± 0.2)°; or
(ii) (6.3 ± 0.2)°, (7.0 ± 0.2)°, (8.9 ± 0.2), (10.9 ± 0.2)° and (11.3 ± 0.2)°.

6. Crystalline form according to claim 1, which is a heminapadisylate salt, and is preferably Form D, which **characterized by** having a powder X-ray diffractogram (PXRD) comprising reflections at 2-Theta angles, when measured at a temperature in the range of from 20 to 30°C with Cu-Kalpha_{1,2} radiation having a wavelength of 0.15419 nm, of:
(i) (8.1 ± 0.2)°, (11.7 ± 0.2)° and (14.2 ± 0.2)°; or
(ii) (8.1 ± 0.2)°, (11.7 ± 0.2)°, (12.7 ± 0.2)° and (14.2 ± 0.2)°; or
(ii) (8.1 ± 0.2)°, (11.7 ± 0.2)°, (12.7 ± 0.2)°, (14.2 ± 0.2)°, and (16.6 ± 0.2)°.

7. Crystalline form of voxelotor napadisylate according to any one of claims 1-4 and 6, **characterized by** comprising about ≤ 1.0 wt.% organic solvent(s).

8. Process for the preparation of crystalline salts of voxelotor napadisylate of any one of claims 1-7, in particular crystalline Form A of voxelotor heminapadisylate according to claims 1 to 3, comprising the following steps:
(i) providing voxelotor;
(ii) adding 1,5-naphthalenedisulfonic acid and at least one organic solvent to obtain a solution or a suspension, wherein 1,5-naphthalenedisulfonic acid and/or the at least one organic solvent contain(s) water;
(iii) optionally seeding the mixture with the crystalline form of voxelotor napadisylate;
(iv) subjecting the mixture provided in (iii) to crystallization conditions leading to the formation of voxelotor napadisylate salt;
(v) optionally isolating the obtained crystalline salt of voxelotor;
(vi) optionally drying the obtained crystalline salt of voxelotor;
(vii) optionally equilibrating the obtained crystalline salt of voxelotor with an atmosphere having a relative humidity of from 25% to 80% at a temperature of from 25°C to 40°C;
(viii) thereby obtaining the crystalline salt of voxelotor napadisylate.

9. Pharmaceutical composition comprising one or more crystalline salts of voxelotor napadisylate of any of claims 1-7, preferably crystalline Form A according to claims 1 to 3 and/or crystalline Form B according to claim 4 and/or crystalline Form D according to claim 6, and one or more pharmaceutically acceptable excipients.

10. Dosage form comprising the pharmaceutical composition as defined in claim 9.

11. Dosage form of claim 10 or pharmaceutical composition of claim 9, for use in a method of treating disorders mediated by hemoglobin and disorders that would benefit from increased tissue oxygenation, preferably, the disorder is stickle cell anemia.
